# EUROPEAN PATENT APPLICATION

(11) **EP 4 706 587 A1**
(43) Date of publication of application: **11.03.2026**
(21) Application number: 24803626.1
(22) Date of filing: 26.04.2024
(51) Int. Cl.: A61C 7/00, A61C 13/00, B33Y 50/00, B33Y 80/00, G16H 50/50, G06T 17/00

(54) **DESIGN METHOD OF TRANSPARENT ORTHODONTIC DEVICE AND ATTACHMENT FOR ORTHODONTICS**

(30) Priority: 05.05.2023 KR 20230058813
(71) Applicant: ODS Co. Ltd, Incheon 21990 (KR)
(72) Inventor: SIM, Miyoung, Incheon 21990 (KR); PARK, Junbeom, Incheon 21974 (KR)
(74) Representative: You Patent
(86) International application number: PCT/KR2024/005668
(87) International publication number: WO 2024/232571

(57) **Abstract**

The method for designing a transparent orthodontic appliance and attachment for tooth alignment according to the present invention has been devised to solve the above technical problems. It is a method for designing an orthodontic appliance using a computer, comprising: a step (S01) of designing the shape of an attachment to be affixed to the surface of a tooth model; a step (S02) of designing the inner surface shape of the transparent orthodontic appliance corresponding to the attachment designed in step S01; and a step (S03) of outputting the transparent orthodontic appliance by means of a 3D printer. The attachment comprises: two pressure surfaces (101) formed to face each other; two inclined surfaces (201) formed to be inclined toward each other while contacting the two pressure surfaces (101); a top surface (301) contacting the edges of the two pressure surfaces (101) and the two inclined surfaces (201); and an attachment surface (401) affixed to the surface of a tooth. This configuration serves as the technical means for solving the problem.

## Description

### Technical Field

The present invention relates to a method for designing a transparent orthodontic appliance and an attachment using a computer. More specifically, it relates to a method for designing a transparent orthodontic appliance and an attachment capable of correcting teeth more efficiently. According to the present invention, not only can the orthodontic effect be enhanced, but also the time and cost required to manufacture the orthodontic appliance can be reduced.

### Background Art

In the field of orthodontics using transparent orthodontic appliances, the present invention relates to a method for designing (by computer) a transparent orthodontic appliance and an attachment in which orthodontic force is more effectively exerted.

Specifically, this involves attaching an attachment (commonly referred to in the industry as an "attachment") to the surface of a tooth, and designing a transparent orthodontic appliance such that a certain force is applied to the attachment, thereby achieving more effective tooth correction. Alternatively, by designing the tooth surface with a groove and designing a transparent orthodontic appliance corresponding to the groove, more effective orthodontic correction can be achieved.

Technology that utilizes attachments affixed to the tooth surface for orthodontic purposes has already been disclosed in Korean Patent Publication No. 10-2148778.

That invention is characterized in that the attachment is affixed not only to the tooth surface but also to the gum surface (root area), thereby guiding the direction of orthodontic force in the desired direction. However, that invention only discloses the direction of orthodontic force applied to the tooth, without suggesting any specific shape of the attachment, and does not disclose the method of forming grooves on the orthodontic tooth model or forming grooves and/or protrusions on the inner surface of the orthodontic appliance. Furthermore, since the appliance is manufactured manually, a significant amount of effort is required, making it very different from the computer-based invention of the present application.

In the process of designing a transparent orthodontic appliance using a computer, the present invention:
designs the attachment affixed to the tooth surface in an optimized form for orthodontics, and designs the inner surface of the transparent orthodontic appliance in a concave shape corresponding to the designed attachment, or
designs the tooth surface with a virtual groove, and designs the inner surface of the transparent orthodontic appliance with a convex ridge corresponding to the groove,
thereby enabling stronger orthodontic force to be effectively exerted on the tooth.

Ultimately, the present invention relates to a program that designs the overall shape of a transparent orthodontic appliance and attachment so that more effective orthodontic force is exerted on the target tooth using a computer.

### Specific Instruction for Implementing the Invention

### Technical Problem

The present invention aims to provide a technical solution whereby an orthodontic appliance (preferably a transparent orthodontic appliance) can be designed quickly and accurately using a computer, thereby enabling more efficient orthodontic treatment of the target tooth with the manufactured appliance.

Another object of the present invention is to provide a design program for an orthodontic appliance in which attachments and/or grooves are installed on the surface of the target tooth so that orthodontic force can be transmitted to the target tooth more effectively.

### Technical Solution

The method for designing a transparent orthodontic appliance and attachment for tooth alignment according to the present invention was devised to solve the above technical problems.

First Embodiment is as folliws.

A method for designing an orthodontic appliance using a computer, comprising: a step(S01) of designing the shape of an attachment to be affixed to the surface of a tooth model,a step(S02) of designing the inner surface of the transparent orthodontic appliance corresponding to the attachment designed in step S01, and a step(S03) of outputting the transparent orthodontic appliance using a 3D printer,
wherein the attachment comprises: two pressure surfaces(101) formed to face each other, two inclined surfaces(201) formed to be inclined toward each other while contacting the two pressure surfaces(101), a top surface(301) contacting the edges of the two pressure surfaces (101) and the two inclined surfaces(201), and an attachment surface(401) to be affixed to the surface of a tooth.

Another Embodiment is as folliws.

A groove(601) is formed either as one groove on a single tooth, or as two grooves on opposite sides of a tooth requiring rotational correction so as to generate a rotational force.

The transparent orthodontic appliance is provided with a protrusion(602) at a position corresponding to the groove(601), the protrusion being formed in a shape corresponding to the groove(601).

### Effects of the Invention

According to the design program of the present invention: An attachment can be affixed to a more accurate position on the target tooth, thereby exerting optimal orthodontic force on the tooth.

A transparent orthodontic appliance corresponding to the attachment or groove can be easily designed and output using a 3D printer, simplifying the manufacturing process and improving cost-effectiveness.

The orthodontic force exerted by the transparent orthodontic appliance can be effectively applied to the target tooth, thereby maximizing orthodontic efficiency while also improving convenience of attachment/detachment and usability.

### Brief Description of the Drawings

FIG. 1: Attachment according to the first embodiment of the present invention.
FIG. 2: Attachment according to the second embodiment of the present invention.
FIG. 3: Attachment according to the third embodiment of the present invention.
FIG. 4 and FIG. 5: Tooth model with the attachment of the present invention affixed.
FIG. 6: Fourth embodiment of the present invention.
FIG. 7: Fifth embodiment of the present invention.
FIG. 8: Sixth embodiment of the present invention.

### Best Mode for Implementing the Invention

The present invention, in a method for designing an orthodontic appliance using a computer, comprises:
a step(S04) of forming a virtual groove(601) on the surface of a tooth model,
a step(S05) of designing the inner surface shape of the transparent orthodontic appliance corresponding to the tooth model designed in step S04, and
a step(S06) of outputting the transparent orthodontic appliance by a 3D printer.

The groove(601) may be formed as one groove on a single tooth, or as two grooves on opposite sides of a tooth requiring rotational correction so as to generate a rotational force.

The inner surface of the transparent orthodontic appliance is formed with a protrusion(602) corresponding to the groove(601).

Wherein the protrusion(602) comprises:
two pressure surfaces(101) formed to face each other, two inclined surfaces(201) formed to be inclined toward each other while contacting the two pressure surfaces(101), a top surface(301) contacting all four edges of the two pressure surfaces(101) and the two inclined surfaces(201), and an attachment surface(401) affixed to the tooth surface.

### Mode for Implementing the Invention

The advantages and features of the present invention, and methods for achieving them, will be apparent from the embodiments described below with reference to the accompanying drawings. However, the present invention is not limited to the disclosed embodiments, but may be implemented in various other forms. The embodiments are provided to fully disclose the present invention and to fully inform those skilled in the art of the scope of the invention, which is defined only by the claims.

The present invention relates to a program for designing a transparent orthodontic appliance using a computer. Here, "computer" includes any device capable of performing computational processing and providing results to the user, including input, processing, and output units.

Images of tooth models in the present specification include multi-dimensional images(2D, 3D, etc.) showing overall tooth arrangement, including medical imaging obtained using tomography. For example, CT (Computed Tomography) images, NMR-CT (Nuclear Magnetic Resonance Computed Tomography) images, PET (Positron Emission Tomography) images, CBCT (Cone-Beam CT) images, and oral scanner images are all included.

The term "target tooth" in the present specification refers to a tooth improperly positioned or abnormally rotated, requiring positional movement and/or rotational correction through orthodontics.

The term "lingual surface" refers to the tooth surface facing the tongue, "labial surface" refers to the surface facing the lips, "buccal surface" refers to the surface facing the inner cheek, "occlusal surface" refers to the upper surface of molars that directly chew food, and "incisal surface" refers to the slanted edge surface of anterior teeth (without occlusal surfaces) that cuts food.

Hereinafter, embodiments of the present invention will be described in detail with reference to the accompanying drawings.

The present invention relates to a method for designing an orthodontic appliance using a computer, comprising: a step(S01) of designing the shape of an attachment to be affixed to the surface of a tooth model; a step(S02) of designing the inner surface shape of the transparent orthodontic appliance corresponding to the attachment designed in step S01; and a step(S03) of outputting the transparent orthodontic appliance by means of a 3D printer.

For designing the orthodontic appliance according to the present invention, an image of the tooth model is displayed. The image of the tooth model is preferably obtained by scanning the patient's oral cavity using a 3D scanner. However, as described above, it is also possible to utilize various types of imaging equipment.

Step S01 is the step of designing the attachment. The attachment is stored in a library in various shapes, from which the attachment most suitable for the target tooth is selected. The selected attachment can be modified on screen in terms of width, height, thickness, etc.

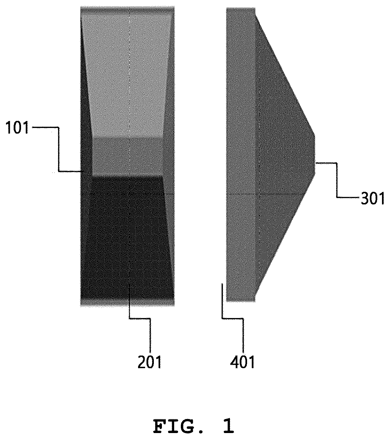

FIG. 1 illustrates an attachment according to the first embodiment of the present invention.

The attachment according to the first embodiment comprises: two pressure surfaces(101) formed to be inclined while facing each other, two inclined surfaces(201) formed to be inclined toward each other while contacting the two pressure surfaces(101), a top surface(301) contacting the edges of the two pressure surfaces (101) and the two inclined surfaces(201), and an attachment surface (401) to be affixed to the tooth surface.

The pressure surface(101) is the portion on which orthodontic force is exerted. When the transparent orthodontic appliance is worn on the target tooth, it applies force to the pressure surface(101), thereby transmitting orthodontic force to the tooth to which the attachment is affixed.

Transparent orthodontic appliances correct teeth by gradually moving them. When the patient wears a transparent orthodontic appliance formed in accordance with the intended final tooth position, the target tooth gradually displaces according to the appliance shape, thereby achieving correction.

When horizontal or vertical movement(including bodily movement, intrusion, extrusion, etc.) is required, the orthodontic force applied directly by the transparent orthodontic appliance has limitations. The attachment of the present invention provides an artificial protrusion on the tooth, enabling orthodontic force to act more effectively.

The transparent orthodontic appliance has an inner surface formed with a concave portion corresponding to the attachment, ensuring that orthodontic force is effectively transmitted to the target tooth.

Although the pressure surface(101) is shown as slightly inclined, the angle of inclination can be adjusted depending on the orthodontic requirements, and in some cases, it may be formed vertically without inclination.

The inclined surfaces(201) are provided on both sides, contacting the two pressure surfaces(101), and are formed to incline toward each other.

While the attachment is essential for effective transmission of orthodontic force, it can hinder insertion and removal of the transparent orthodontic appliance. For ease of insertion and removal, the inclined surfaces(201) are formed at a certain angle.

The top surface(301) is formed to contact the edges of the two pressure surfaces(101) and the two inclined surfaces(201). The width of the top surface(301) may be adjusted, and the width/angle of the pressure surfaces(101) and inclined surfaces(201) can also be modified accordingly.

The attachment surface(401) is the portion that comes into contact with and is affixed to the tooth surface. Although not shown in the drawings, the attachment surface(401) may be formed with the same curvature as the tooth surface, thereby improving adhesion.

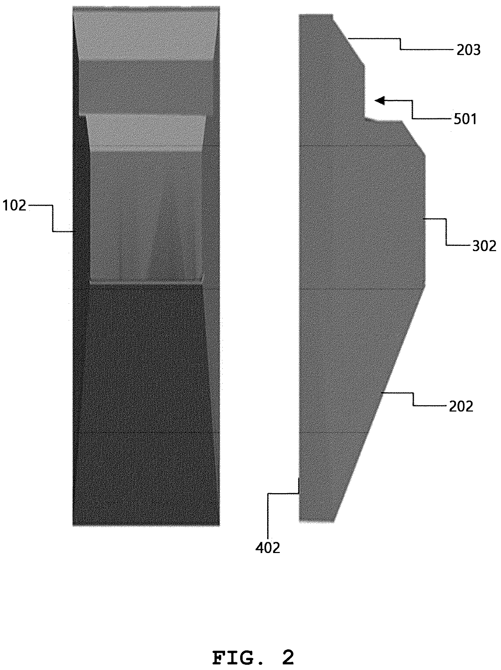

FIG. 2 illustrates an attachment according to the second embodiment of the present invention. The attachment of the second embodiment is specifically designed for extrusion correction. Although only the maxillary case is illustrated, in the mandibular case the orientation of the attachment would be reversed, while the principle remains the same.

The attachment according to the second embodiment comprises: two pressure surfaces(102) formed to face each other, a first inclined surface(202) and a second inclined surface(203), each formed to be inclined toward each other while contacting the two pressure surfaces(102), a step portion(501) formed on the second inclined surface(203), a top surface(302) contacting the edges of the pressure surfaces (102), the first inclined surface(202), and the second inclined surface (203), and an attachment surface(402) to be affixed to the tooth surface.

The configuration of the two pressure surfaces(102), the first inclined surface(202), and the second inclined surface (203) is essentially the same as described for the first embodiment, and is therefore omitted here.

The step portion(501) is provided on the second inclined surface (203) such that the transparent orthodontic appliance catches on it. When the appliance is worn, it engages the step portion (501), allowing more effective orthodontic force (particularly vertical force for extrusion correction) to be exerted on the target tooth. This effective orthodontic force refers to a more continuous and stronger corrective force.

The first inclined surface(202) is angled to facilitate insertion of the appliance, while the step portion(501) on the second inclined surface (203) provides a catch for applying effective orthodontic force to the tooth. Like the first embodiment, horizontal corrective force may also be exerted via the pressure surfaces(102).

To detach the appliance, the user may simply pull outward at the edge of the appliance, easily removing it from the attachment.

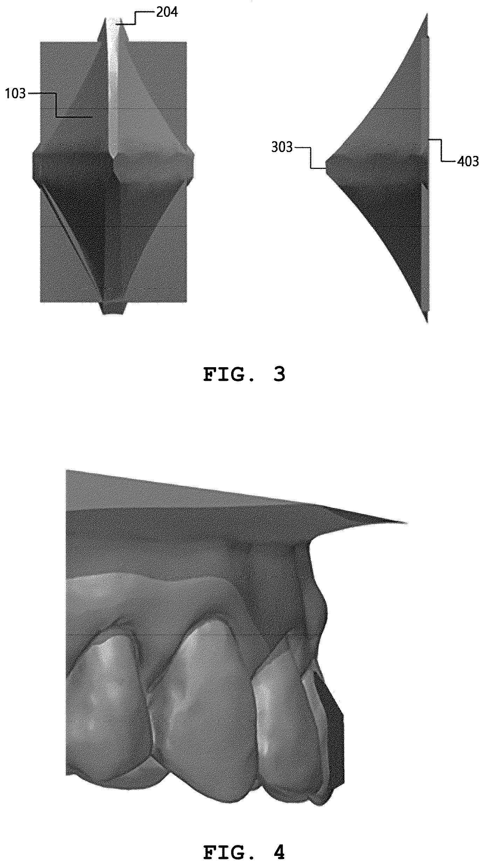

FIG. 3 illustrates an attachment according to the third embodiment of the present invention.

The attachment comprises: two triangular pressure surfaces (103) facing each other, two inclined surfaces (204) formed to be inclined toward each other while contacting the two pressure surfaces (103), a top surface (303) contacting the edges of the two pressure surfaces (103) and the two inclined surfaces (204), and an attachment surface (403) to be affixed to the tooth surface.

The pressure surfaces (103) are triangular in shape and face each other. The inclined surfaces (204) contact the pressure surfaces (103) and are inclined toward each other.

Although the pressure surfaces (103) are shown slightly inclined, the inclination may be adjusted depending on orthodontic requirements, and may even be vertical without inclination.

Although not shown in FIG. 3, a step portion (501) may also be added to the attachment according to the third embodiment. By installing a step portion (501) on the inclined surface (204), it may be used for extrusion correction or to prevent the appliance from easily disengaging from the tooth.

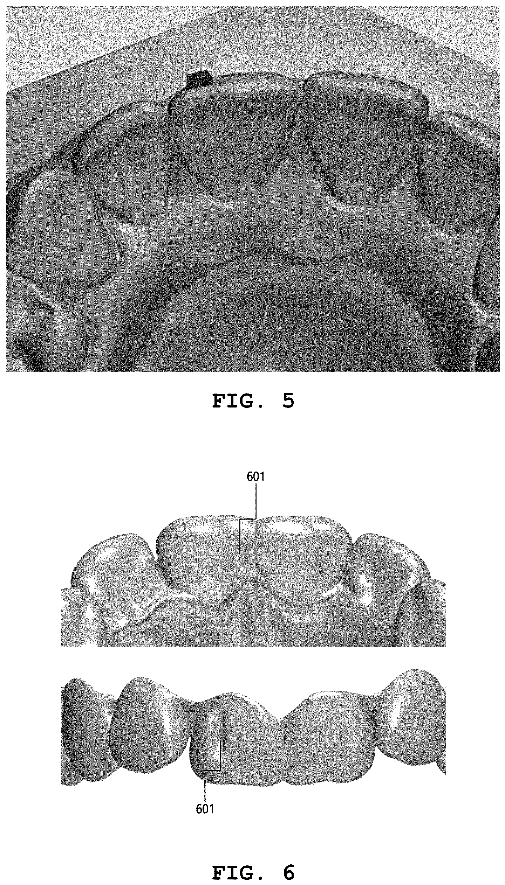

FIGS. 4 and 5 illustrate an attachment (in blue) affixed to a tooth model according to the present invention, shown in side view and top view respectively.

The invention has thus far been described in relation to attachments affixed to target teeth. However, attachments according to the invention may also be affixed to non-target teeth. For example, when lateral movement of the target tooth is desired, attachments may be placed on adjacent teeth, using them as anchoring points. The transparent orthodontic appliance supported by attachments on adjacent teeth can then exert stronger corrective force on the target tooth.

Accordingly, attachments may be affixed not only to target teeth but also to non-target teeth. The anchor teeth may be directly adjacent or separated by one or more teeth depending on oral conditions.

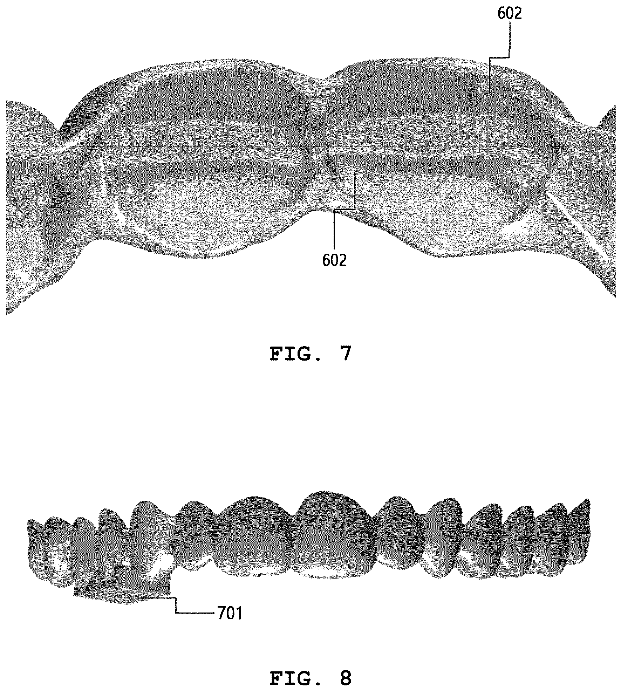

Figures 6 and 7 illustrate a fourth embodiment and a fifth embodiment, respectively, according to the present invention.

FIG. 6 illustrates a fourth embodiment in which an artificial groove (601) is formed on one side surface(lingual or labial) of a target tooth in the tooth model. FIG. 7 illustrates a fifth embodiment in which grooves(601) are formed on both side surfaces, with corresponding protrusions(602) formed on the inner surface of the transparent orthodontic appliance.

When a groove(601) is formed in the tooth model, a corresponding protrusion(602) is formed on the appliance. The protrusion enhances orthodontic force exerted on the target tooth.

The location of the groove(601) is determined based on tooth shape and the desired direction of orthodontic force, while its size is adjusted according to the required corrective force.

Multiple grooves may be formed for a single tooth. In particular, when two grooves(601) are formed on opposite sides of a tooth, rotational correction can be effectively achieved.

By applying orthodontic force from opposite sides, rotational moment is generated to induce tooth rotation.

Although FIGS. 6 and 7 show a specific groove shape, the groove (601) may vary, for example, as a dimple or as an elongated groove.

The corresponding protrusions(602) formed in the appliance will match the groove shapes and reinforce orthodontic force.

The protrusions(602) may also have the same shapes as the attachments described in the first to third embodiments, in which case the grooves(601) in the tooth model will correspond to the negative shapes of those attachments.

Thus, the first to third embodiments involve forming protrusions on the tooth model, while the fourth and fifth embodiments involve forming grooves(601), resulting in corresponding protrusions(602) on the appliance.

The shapes and sizes of grooves(601) and protrusions(602) may vary depending on orthodontic conditions, without departing from the scope of the invention.

IG. 8 illustrates a sixth embodiment relating to a design method for an orthodontic appliance that utilizes occlusal force to maximize corrective force.

For a tooth requiring intrusion correction, force must be applied in the gingival direction. However, transparent orthodontic appliances structurally have limitations in applying intrusion force.

In this embodiment, a horizontal block (701) is formed on the upper outer surface of the appliance (occlusal surface in the case of molars) in contact with the tooth requiring intrusion.

When the patient chews while wearing the appliance, force is naturally applied to the horizontal block (701), which in turn applies force to the tooth requiring intrusion correction. Thus, the patient's masticatory movement contributes effectively to intrusion correction.

The embodiments and effects described above are merely examples and do not limit the claims of the invention. Various modifications and alterations may be made without departing from the technical spirit of the invention, and such simple design changes fall within the technical scope of the present invention.

## Claims

1. A method for designing an orthodontic appliance using a computer, comprising:
a step (S04) of forming a virtual groove (601) on the surface of a tooth model;
a step (S05) of designing the inner surface shape of the transparent orthodontic appliance corresponding to the tooth model designed in step S04; and
a step (S06) of outputting the transparent orthodontic appliance by means of a 3D printer,
wherein the groove (601) is formed either as one groove on a single tooth, or as two grooves on opposite sides of a tooth requiring rotational correction so as to generate a rotational force,
wherein the inner surface of the transparent orthodontic appliance is formed with a protrusion (602) at a position corresponding to the groove (601),
and wherein the protrusion (602) comprises:
two pressure surfaces (101) formed to face each other;
two inclined surfaces (201) formed to be inclined toward each other while contacting the two pressure surfaces (101);
a top surface (301) contacting all four edges of the two pressure surfaces (101) and the two inclined surfaces (201); and
an attachment surface (401) affixed to the surface of a tooth,
whereby a method for designing a transparent orthodontic appliance and attachment for tooth alignment is provided.

2. A method for designing an orthodontic appliance using a computer, comprising:
a step (S04) of forming a virtual groove (601) on the surface of a tooth model;
a step (S05) of designing the inner surface shape of the transparent orthodontic appliance corresponding to the tooth model designed in step S04; and
a step (S06) of outputting the transparent orthodontic appliance by means of a 3D printer,
wherein the groove (601) is formed either as one groove on a single tooth, or as two grooves on opposite sides of a tooth requiring rotational correction so as to generate a rotational force,
wherein the inner surface of the transparent orthodontic appliance is formed with a protrusion (602) at a position corresponding to the groove (601),
and wherein the protrusion (602) comprises:
two pressure surfaces (102) formed to face each other;
a first inclined surface (202) and a second inclined surface (203), both formed to be inclined toward each other while contacting the two pressure surfaces (102);
a step portion (501) formed on the second inclined surface (203);
a top surface (302) contacting all four edges of the two pressure surfaces (102), the first inclined surface (202), and the second inclined surface (203); and
an attachment surface (402) affixed to the surface of a tooth,
whereby a method for designing a transparent orthodontic appliance and attachment for tooth alignment is provided.
